# EUROPEAN PATENT APPLICATION

(11) **EP 0 827 724 A2**
(43) Date of publication of application: **11.03.1998**
(21) Application number: 97115149.3
(22) Date of filing: 02.09.1997
(51) Int. Cl.: A61F 2/00

(54) **Prosthesis for hernioplasty with preformed monofilament polypropylene mesh**

(30) Priority: 09.09.1996 CH 2188/96
(71) Applicant: Herniamesh S.r.l., 27100 Pavia (IT)
(72) Inventor: Trabucco, Ermanno, Greatcrech, Long Island, NY 11020 (US)
(74) Representative: Moretti, Francesco, Dr.

(57) **Abstract**

A prosthesis for hernioplasty made of a precured monofilament polypropylene mesh of surgical quality and controlled memory, thermally stable and biocompatible, is preformed into the shape of a nail (1) and has a median hole (2) for the passage of the spermatic cord, which is possible with the slit (3) connected to said hole. The optimal rigidity, the absence of the memory typical of the known PPM, the roughness allow a perfect adhesion and fit of the mesh with the contact surfaces with no tendency to crumple. Porosity and monofilament allows a quick fibroplastic infiltration avoiding possible infections; this increases also the mechanical adhesion for the tough reaction of incorporation of the newly formed connective tissue into the mesh, avoiding any displacement and allowing its tension free sutureless implant, so as to avoid complications after the operation and relapses unlike the prostheses of the prior art. Such a prosthesis allows an operation in outpatient way without stay, resuming the normal current home - work activity after few hours and the complete physical recovery without hospitalization after about one week, with great advantages from the social-economic point of view, because of saving the hospitalization costs and avoiding to lose working days. This prosthesis finds particular application in surgical repair of herniae, more particularly in the hernioplasty according to Trabucco's technique.

## Description

The invention relates to a preformed prosthesis for hernioplasty.

The hernia problem has a great importance from the social as well as economic point of view. Indeed it is a pathological condition affecting predominantly men (9:1 compared to women) at every age, but preferably in the working age (60% of all herniae occur between 45 and 60 years).

With the surgical techniques previously used, the operation required hospitalization of 5 to 12 days according the the organization and efficiency of the medical facilities. The working rehabilitation of the patient was slow and hampered by an objective difficulty ad limitation of recovery, with the result of a secondary social damage due to the impossibility of enploying workers for a period of about 30-60 days.

With the present invention the therapeutic course is totally revolutionized, with possibility of discharge of the patient immediately after the operation, that can now be performed with local anaesthesia. As the patient is fully self-sufficient, he can undergo outpatient medical checks and in about one week he is absolutely able to resume any working activity without limitations, while he may resume secondary activities even the day after the operation.

It is a peculiar advantage of the present invention, besides the possibility of carrying out an original, simple and effective surgical technique, also or even above all, the possibility of creating a new methodological approach to the hernial pathology with the criteria of the "Day Surgery", that is extremely developed in the United States of America and is now being spread all over the world, namely surgical operations carried out in non-hospital medical facilities, specifically and suitably equipped from the surgical, anaesthetic and paramedical point of view, to carry out even multispecialized surgery not requiring post-operative stay as it is performed on patients in condition to look after themselves. Medical facilities were created in the U.S. and spread worldwide, which are surgically autonomous, specifically equipped and suitably organized to offer the patients comfort and a considerable reduction of medical costs (it is to be pointed out that one day of hospitalization nowadays costs about $ 1,500 in the U.S., ITL 600,000 to 800,000 in Italy, about CHF 1,000 in Switzerland).

In the U.S. about 500,000 operations of inguinal hernioplasties out of about 800,000 new diagnoses are effected every year. This means that there is a rather high percentage of patients, who are trusting to the efficiency of the surgical therapy and undergo the surgery as soon as the affection is diagnosed. For instance in Italy the number of operations for hernioplasties may be estimated of about 125,000 a year, although there are no updated statistics. The total amount of surgeries for inguinal hernia seems however to be increased of about 20% in the last two years following the achievement of the surgical methods using alloprosthesis. Therefore also in Italy arid generally in Europe occurs the phenomenon already noticed in the U.S. of the increase of new patients stimulated to the surgery as a result of trusting the new methods.

It is to be noted that simplicity, rapidity and efficiency of the method using parietal prostheses allowed in any case the surgeon to broaden the indications to surgery also to patients who could not undergo a surgical treatment for a serious situation of the general state of health.

All over the world the surgical technique still more frequently used is the direct tissutal suture (Bassini-Shouldice) spread in every country since many years with very variable results and a percentage of 3% to 15% relapses in the various records of cases. It was however noted recently the progressive diffusion of techniques using parietal prostheses of the type MARLEX-PROLENE, with operations generally carried out in outpatient way with local anaesthetic and a negligible (0.2%) relapse rate (Trabucco-Lichtenstein).

The original method disclosed by the Inventor using the present invention, consists of the arrangement on the posterior plane of the inguinal canal of a prosthesis made of a polypropylene mesh making a durable and resistant reinforcement of the whole inguinal region. The mesh is positioned in a closed anatomic space where no suture stitch is required in view of the particular shape of the prosthesis, so that it is totally tension free, as the tension between prosthesis and tissue is the main relapse reason.

According to the prior art, this reinforcement was previously obtained with a hernial plastic suture of the lower edge of the abdomen broad muscles with the reflex part of the inguinal ligament. On the muscle fascial suture seam, in a manner varying from one technique to the other and from one patient to the other, a tissular tension was built, which is considered to be the cause of the collapse, even after years, of the hernioplasties and the appearance of a hernial relapse with a more difficult and complex surgical approach and treatment.

With the prosthesis being the object of the present invention, a polypropylene mesh is infiltrated with fibroplasia immediately after the operation, and immobilized after some weeks by deposition of collagen. The unit consisting of mesh and fibroplastic reaction forms a hernia restraint of a huge strength making a relapse almost impossible.

Recent preliminary statistics obtained by the Inventor and his school during the research and development stage, allow to foresee a relapse rate well under 02.%.

The polypropylene prosthesis (polypropylene mesh, hereinafter PPM) is the material preferred by surgeon for the modern hernial surgery.

PPM shows high tensile strength, is biologically stable, produce a quickier fibroplastic proliferation, bringing to a more efficient collagen deposition, as there is no evidence of the possibility of producing allergic responses in polypropylene (PP) or of any carcinogenic character.

PPM is very resistant to infection just for its monofilamentary nature; however it has also some disadvantages such as memory and thermolability at the temperature of autoclave sterilization of 126°C, at which the mesh undergoes a deformation, recalling that PP has a melting point of about 165°C.

A memory holding mesh is difficult to be implanted, as it has the tendency to crumple and to place itself in an incorrect way, and this malpositioning may occur either during or after the implant, causing dead spaces, serous formations, mesh migration and relapses.

A PPM retaining a surface memory and resisting such a tendency to crumple is called hereinafter controlled memory (CM) mesh.

A PPM resisting deformation after sterilization with ethylene oxide and retaining its features after implant is called hereinafter surgical quality (SQ) mesh.

Nowadays it is possible to find on the market three PPM products, known as Marlex (Bard), Prolene (Ethicon) and Trelex (Meadox). These mesh articles have similar monofilament thickness and textile characteristics, and show the above mentioned merits and defects of the PPM types of the prior art.

The development of hernial surgery and the techniques recently introduced, cause the need to put at disposal of the surgeon mesh having the above indicated features of biocompatibility, surgical quality (SQ) and controlled memory (CM), with a broad range of porosity and texture and with performed shapes, such as the mesh of the present invention.

These features can improve the prosthesis biocompatibility and make its implantation easier. Indeed the higher is the mesh porosity and quickier is the fibroplastic infiltration and therefore higher the resistance to infections.

In order to obtain the ideal rigidity, a correct proportion between monofilament thickness and pore size must be present, as the lower is the filament thickness and the lower is the mesh rigidity. Also the texture quality affects the rigidity of the article. These and other considerations are taken into account in the present invention. More particularly the present invention relates to a hernioplasty prosthesis, characterized by comprising a mesh of precured monofilament polypropylene, performed to an anatomic predetermined shape. In one preferred embodiment, said mesh is preformed, premolded in the shape of a nail, with a median hole connected to a longitudinal slit.

The hernioplasty prosthesis according to the present invention is characterized by the fact of being of surgical quality (SQ), namely with high porosity, wider weave, monofilament and mesh treated after weaving to increase the thermal stability, namely heat resistance by preheating and pretensioning said monofilament and mesh.

The hernioplasty prosthesis according to the present invention is also characterized by comprising a single layer and having controlled memory (CM) by preheating and pretensioning, to obtain the ideal rigidity and roughness by means of a correct proportion of monofilament thickness, pore size, texture to allow its perfect fit to the contact surfaces and increase its mechanical adhesion avoiding any crumpling possibility. The monofilament is treated to modify the memory and prevent the tendency of the mesh to crumple. Implantatin is therefore easier and possible complications are avoided.

All the mesh articles are supplied ready for use with controlled flat memory. These characteristics of rigidity, roughness and memory allow the prosthesis according to the present invention to be free of sutures as it is totally tension free, so that it is free to fit perfectly to the surfaces of its contact.

In order to obtain SQ and CM, PPM undergoes special procedures without chemical treatments before the weaving phase. Achievement of these qualities allows to obtain really innovative mesh. The mesh is preformed rigid or semirigid for tension free sutureless use. Thus the indication to use a double mesh is overcome, which was dictated by the need of having at disposal an article having no tendency to crumple during the implantation procedures. Such a mesh was originally made in a craftsmanlike way by joining two polypropylene mesh pieces, shaping them, welding the edges and then gas sterilizing them.

A double mesh produced in this way had however the disadvantage of a deed space between the two mesh pieces, namely a barrier showing the fibroplastic infiltration, causing the prosthesis to be less resistant to infections. It was proved that the new mesh according to the present invention with SQ and CM, has characteristics of elasticity and strength higher than the double layer, as its surface memory allows to obtain an implant that does not crumple during the surgical procedures.

The present invention relates also to a plug for hernioplasty characterized by comprising a shaped mesh of monofilament polypropylene, with SQ and CM, which is folded along its geometrical axes in the form of a dart. A macroporous mesh is preferably used to make the plug. Said dart-shaped plug is particularly adapted for the treatments at the inner inguinal ring. After the application the plug opens so as to create a better protection in the space behind the fascia-trasversalis, thus preventing the relapse of the sac hernial formation. This mesh is also indicated for treating laparocele or inguinal hernioplastics with the Lichtenstein or Rives technique.

The present invention relates also to the use of the prosthesis according to the invention in the surg ry and repair of different kinds of herniae of the abdominal and thoracic wall and in the surgery and treatment of herniae and hernioplasties according to Trabucco.

Hereinafter in an exemplary and non limiting way a description is given of a particular embodiment of the article according to the present invention, reference being also made to figures of the accompanying illustrative drawings, in which:
Fig. 1 shows the prosthesis for hernioplasty surgery in the form of a nail in its standard size;
Fig. 2 shows the mesh folded in various ways for the preparation of the dart-shaped plug;
Fig. 3 shows the positioning of the nail-shaped mesh on the posterior wall of the inguinal canal; and
Fig. 4 shows the dissection and invagination of the direct sac with the plug implant.

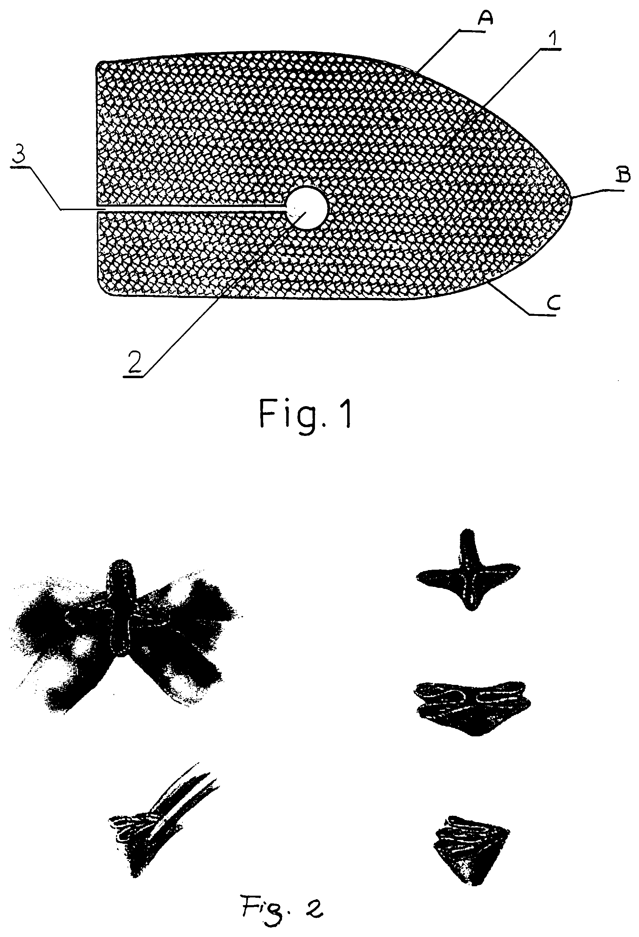

The prosthesis in the shape of a nail 1 (Fig. 1) is not cut every time it is required, but it is of a standard size: on the basis of the intraoperational measurements it was observed that in most cases there are no noticeable differences in the distance between the upper front iliac spine, deep inguinal ring and pubic tubercle.

The prosthesis (Fig. 1) for hernioplasty surgery according to the present invention, characterized by its preformed shape of a nail 1, is of standard size, having the following measures:
length 10 cm; height 4.5 cm; the hole 2 for passage of the funiculus is arranged at 6 cm from the medial end and at 1.5 cm from the lower edge; diameter of the hole 1.0 em; lenght of the longitudinal slit 3 is 3.5 cm.

Another embodiment of the standard prosthesis has the following measures:
length 10 cm; height 4.5 cm; the hole 2 for the passage of the funiculus is arranged at 6 cm from the medial end and at 1.5 cm from the lower edge; diameter of the hole 1.2 cm; length of the longitudinal slit 3 is 3.4 cm.

For both embodiments it is very important the bend radius of the nail point, calculated at the points A, B and C indicated in Fig. 1, namely A= 7 cm ; B= 1,5 cm ; C= 6,5 cm.

The plug 4 for hernioplasty surgery (Fig. 2) is obtained from a monofilament polypropylene mesh, with SQ and CM, preformed as a circle with a diameter of 5 cm or a square with a 5 cm side, folded along its geometrical axes, orthogonal or diagonal diameters, respectively, in the form of a dart. Both these types of prosthesis have the advantage, in comparison with the prior art, to be more rigid, not to be corrugated, even if they are not fixed at all, and to lose the plastic memory which is typical of polypropylene.

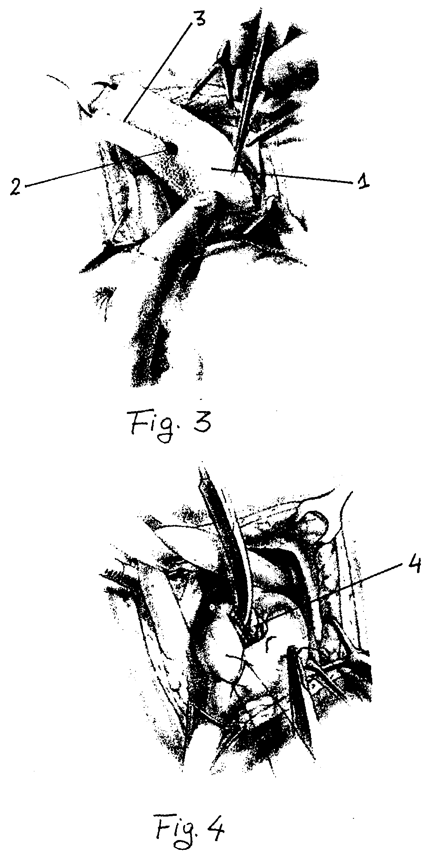

The Inventor of the present invention modified also the approach to the outer oblique hernia with the prosthesis according to the present invention; after having isolated the sac from the funiculus elements and retroflexed said sac, he applies inside the inguinal ring opened through the side a plug 4 (Fig 4) made of polypropylene in the form of a dart, which is then fixed with a stitch like a tobacco pouch. As already mentioned, the plug 4 is made with a prosthesis of a generally square form with 5 cm side, which is folded along its diagonals with a polypropylene cross-stitch (Fig. 2). In presence of a direct hernia, the posterior wall of the inguinal wall, which is opened though the side, is introflexed tension free, with a continuous pinned double suture extending from the pubis tubercle to the deep inguinal ring.

If the defect is very wide (Fig. 3) and the posterior wall is much damaged, a preperitoneal prosthesis is applied and fixed to the wall with spaced stitches. Then the preformed prosthesis 1 is applied (Fig. 1) under the fascia of the exterior oblique muscle; its medial edge must protrude for at least 1 cm from the pubis tubercle and its inferior margin must lean on the concavity of the inguinal ligament, while the superior margin must lean on the anterior sheath of the abdominal rectum and in the interior oblique muscle (Fig. 3). The prosthesis 1 is not fixed in any way; one stitch only closes laterally the slit 3 of the hole 2, for the passage of the spermatic cord (Figs. 1 and 3). Without suture hematomata and accidental trapping of the sensory nerves of the region are avoided; moreover the prosthesis is free to fit perfectly to the surfaces of contact, so as to avoid excessive tensions and formation of dead spaces. The mechanical adhesion of the prosthesis to the surrounding structures, made possible by the rough surface, and the tough reaction of incorporation by the newly formed connective tissue, hinder the mesh displacement.

The fascia of the exterior oblique muscle is then reconstructed above the mesh and under the spermatic funiculus, thus remaining in the subcutaneous tissue, through two continuous sutures; the one extends from the pubis tubercle to the new superficial inguinal ring, located in correspondence with the deep ring, and the other extends aside the spermatic cord.

With the prosthesis of the invention it is now possible according to the Trabucco's technique, to carry out the operation in outpatient way without needing further stay, as the patient can resume his ordinary activity about two hours after the operation (current home, work activity) and the physical activity with strain approximately one week thereafter.

This involves a double advantage from the economic-social point of view: saving high hospitalization costs for the absence of stay on the one hand, and immediate return to work, resuming the normal working activity without losing further working days, on the other hand.

## Claims

1. Prosthesis for hernioplasty, characterized by comprising a mesh made of cured monofilament polypropylene, preformed into a predetermined anatomical shape.

2. Prosthesis for hernioplasty according to Claim 1, characterized in that the monofilament polypropylene mesh is preformed in the shape of nail (1) with median hole (2) connected to a longitudinal slit (3) (Fig. 1).

3. Prosthesis for hernioplasty according to Claim 2, characterized in that the preformed nail shape (1) is premolded and has standard dimensions with the following measures:
length 10 cm; height 4.5 cm; hole (2) for the passage of the funiculus at 6 cm from the medial end and at 1.5 cm from the lower edge; diameter of the hole 1.0 cm; length of the longitudinal slit (3) 3.5 cm (Fig.1).

4. Prosthesis for hernioplasty according to Claim 2, characterized in that the preformed nail shape (1) is premolded and has standard dimensions with the following measures:
length 10 cm; height 4.5 cm; hole (2) for the passage of the funiculus at 6 cm from the medial end and at 1.5 cm from the lower edge; diameter of the hole 1.2 cm; length of the longitudinal slit (3) 3.4 cm and bend radii A= 7 cm; B= 1,5 cm; C= 6,5 cm (Fig. 1).

5. Prosthesis for hernioplasty according to any of the preceding claims, characterized in that it is of surgical quality (SQ), namely with high porosity for a quick fibroplastic infiltration, and thermally stable, namely heat resistant by preheating and pretensioning the monofilament and the mesh.

6. Prosthesis for hernioplasty according to any of the preceding claims, characterized in that it consist of a single layer and has a controlled memory (CM) obtained by preheating and pretensioning, to obtain the ideal rigidity and roughness, by a correct proportioning of monofilament thickness, pore size, texture so as to allow its perfect fit to the contact surfaces and increase its mechanical adhesion without tendency to crumple.

7. Prosthesis for hernioplasty according to any of the preceding claims, characterized in that it is totally tension free sutureless, thus being free to fit perfectly to the surfaces of contact.

8. Prosthesis for hernioplasty according to Claims 5, 6 and 7, characterized by being perfectly biocompatible so as to avoid practically any relapse with the SQ and CM biocompatibility criteria.

9. Plug for hernioplasty according to Claims 1, 5, 6, 7 and 8, characterized by comprising a shaped mesh of monofilament polypropylene with surgical quality and controlled memory, folded along its geometrical axes in the form of a dart (Fig. 2).

10. Plug for hernioplasty according to Claim 9, characterized in that the mesh has a round shape and a standard size of 5 cm. diameter.

11. Plug for hernioplasty according to Claim 9, characterized in that the mesh has a square shape and a standard size with a 5 cm side.

12. Use of the prosthesis for hernioplasty according to any of Claims 1 to 8, in surgery and treatment of herniae of the abdominal wall, more particularly of inguinal hernioplasty according to the Trabucco's technique (Fig. 3).

13. Use of the plug according to any of Claims 9, 10, or 11, for tamponage, closure and invagination of the sac in the treatment and surgery of hernioplasty with the Trabucco's technique, more particularly in repairing crural herniea and inguinocrural relapses (Fig. 4).

14. Use of the plug according to any of Claims 9, 10 or 11, together with the hernial prosthesis according to any of Claims 1 to 8, in the treatment and surgery of herniae of the abdominal and thoracic wall.
